(19) Europäisches Patentamt
European Patent Office
Office européen des brevets

(11) **EP 3 714 918 A1**

(12) **EUROPEAN PATENT APPLICATION**

(43) Date of publication:
**30.09.2020 Bulletin 2020/40**

(51) Int Cl.:
***A61M 1/16*** (2006.01)     ***A61M 1/36*** (2006.01)
***B01J 20/34*** (2006.01)

(21) Application number: **20164524.9**

(22) Date of filing: **20.03.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.03.2019 US 201916369817**

(71) Applicant: **Medtronic, Inc**
**Minneapolis, MN 55432 (US)**

(72) Inventors:
- **COLLIER, Kenneth J.**
  **Dellwood, MN 55110 (US)**
- **HOBOT, Christopher M.**
  **Rogers, MN 55374 (US)**
- **GERBER, Martin T.**
  **Maple Grove, MN 55369 (US)**
- **PUDIL, Bryant J.**
  **Plymouth, MN 55447 (US)**

(74) Representative: **Maschio, Antonio**
**Maschio & Soames IP Limited**
**30 Carlton Crescent**
**Southampton SO15 2EW (GB)**

(54) **PRECISION RECHARGING USING PATIENT PRE-DIALYSIS BUN LEVELS**

(57)    The invention relates to devices, systems, and methods for recharging zirconium phosphate. The devices, system, and methods use a patient pre-dialysis BUN level to set one or more recharge parameters for recharging the zirconium phosphate. The devices, systems, and methods allow for precision recharging of the zirconium phosphate based on the patient pre-dialysis BUN level.

FIG. 4

**Description**

CROSS-REFERENCE TO RELATED APPLICATIONS

This application is a continuation in part of U.S. Patent Application No. 16/148,383 filed October 1, 2018, which claims benefit of and priority to U.S. Provisional Application No. 62/583,356 filed November 8, 2017.
Also, this application is a continuation in part of U.S. Patent Application No. 15/872,363 filed January 16, 2018, which claims benefit of and priority to U.S. Provisional Application No. 62/456,700 filed February 9, 2017, and the disclosures of each of the above-identified applications are hereby incorporated by reference in their entirety.

FIELD OF THE INVENTION

The invention relates to devices, systems, and methods for recharging zirconium phosphate. The devices, system, and methods use a patient pre-dialysis BUN level to set one or more recharge parameters for recharging the zirconium phosphate. The devices, systems, and methods allow for precision recharging of the zirconium phosphate based on the patient pre-dialysis BUN level.

BACKGROUND

Zirconium phosphate is used in sorbent dialysis to remove waste and unwanted solutes including ammonium, potassium, calcium, and magnesium ions from dialysate. After use the zirconium phosphate can be reprocessed or recharged to restore functional capacity of the material. The amounts of recharge solutions necessary to recharge the zirconium phosphate depend in part on the amount of ammonia removed by the zirconium phosphate during therapy, which in turn depends on the patient pre-dialysis BUN level. Known systems and methods are unable to set recharge parameters used in recharging zirconium phosphate based on the patient pre-dialysis BUN level and instead use a one size fits all approach, using enough recharge solutions to ensure complete recharging of the zirconium phosphate for patients with the highest BUN levels. In most cases, the one size fits all approach uses more of the recharge solutions than necessary, driving up costs and waste and increasing the time necessary for recharging.
Hence, there is a need for precision sorbent material recharging systems and methods that use the patient pre-dialysis BUN level to set the recharge parameters. The need extends to performing precision sorbent recharging accurately, efficiently, and economically. The need includes systems and methods that allow for such precision recharging without the need for excess chemicals or time.

SUMMARY OF THE INVENTION

The first aspect of the invention relates to a system. In any embodiment, the system can comprise a recharging flow path comprising one or more recharge solution sources; the one or more recharge solution sources fluidly connectable to an inlet of a zirconium phosphate sorbent module containing zirconium phosphate; and at least one pump for introducing one or more recharge solutions from the one or more recharge solution sources through the zirconium phosphate sorbent module; and a processor, the processor programmed to set one or more recharge parameters to recharge the zirconium phosphate within the sorbent module based on a patient pre-dialysis BUN level.
In any embodiment, the one or more recharge solution sources can comprise a brine source and a water source.
In any embodiment, the one or more recharge parameters can comprise at least one of: time of recharging, recharge temperature, volume of at least one recharge solution, concentration of at least one recharge solution, and combinations thereof.
In any embodiment, the processor can set a volume of the one or more recharge solutions using an equation volume = A * Pre-BUN + B; wherein and B are based on patient pre-dialysis solute level and a concentration of the one or more recharge solutions.
In any embodiment, the processor can be programmed to set the volume of the one or more recharge solutions based on the patient pre-dialysis BUN level and a concentration of the one or more recharge solutions.
In any embodiment, the processor can be programmed to set the concentration of the one or more recharge solutions based on the patient pre-dialysis BUN level, and a volume of the one or more recharge solutions to be used.
In any embodiment, the system can comprise at least one ammonia sensor in an effluent line fluidly connectable to an outlet of the zirconium phosphate sorbent module; wherein the processor is programmed to estimate the patient pre-dialysis BUN level based on an ammonia concentration in an effluent from the zirconium phosphate sorbent module.
In any embodiment, the processor can be programmed to estimate the patient pre-dialysis BUN level based on a comparison of the ammonia concentration in the effluent with a known ammonia measurement curve.
In any embodiment, the processor can be programmed to receive a patient pre-dialysis BUN level from a user.

In any embodiment, the system can comprise a reader in communication with the processor, the reader receiving the patient pre-dialysis BUN level from a readable component on or in the sorbent module.

The features disclosed as being part of the first aspect of the invention can be in the first aspect of the invention, either alone or in combination, or follow a preferred arrangement of one or more of the described elements.

The second aspect of the invention is drawn to a method. In any embodiment, the method can comprise the steps of recharging zirconium phosphate within a zirconium phosphate sorbent module by introducing one or more recharge solutions from one or more recharge solution sources through the zirconium phosphate sorbent module with one or more recharge parameters; wherein the one or more recharge parameters are set based on a patient pre-dialysis BUN level.

In any embodiment, the one or more recharge parameters can comprise at least one of: time of recharging, recharge temperature, volume of at least one recharge solution, concentration of at least one recharge solution, and combinations thereof.

In any embodiment, the one or more recharge solution sources can comprise a brine source and a water source.

In any embodiment, the method can comprise the step of estimating the patient pre-dialysis BUN level based on an ammonia sensor in an effluent line fluidly connected to an outlet of the zirconium phosphate sorbent module.

In any embodiment, the method can comprise the step of estimating the patient pre-dialysis BUN level based on one or more sensors in a dialysate flow path.

In any embodiment, the method can comprise the step of receiving the patient pre-dialysis BUN level from a user.

In any embodiment, setting the recharge parameters can comprise setting the concentration of the one or more recharge solutions based on the patient pre-dialysis BUN level and a volume of the one or more recharge solutions to be used.

In any embodiment, setting the recharge parameters can comprise setting the volume of the one or more recharge solutions based on the patient pre-dialysis BUN level and a concentration of the one or more recharge solutions.

In any embodiment, setting the recharge parameters can comprise setting the volume of the one or more recharge solutions using an equation volume = A * Pre-BUN + B; wherein A and B are based on patient pre-dialysis levels of one or more solutes, dialysis session parameters, a concentration of the one or more recharge solutions, and recharge parameters.

In any embodiment, the method can be performed by a processor of a sorbent recharger.

The features disclosed as being part of the second aspect of the invention can be in the second aspect of the invention, either alone or in combination, or follow a preferred arrangement of one or more of the described elements.

## BRIEF DESCRIPTION OF THE DRAWINGS

FIG. 1 shows a sorbent recharger.
FIG. 2 shows a zirconium phosphate recharging flow path.
FIG. 3 shows a dialysate flow path with sensors capable of determining a patient pre-dialysis BUN level.
FIG. 4 is a flow chart illustrating a method of precision recharging based on a patient pre-dialysis BUN level.

## DETAILED DESCRIPTION OF THE INVENTION

Unless defined otherwise, all technical and scientific terms used have the same meaning as commonly understood by one of ordinary skill in the art.

The articles "a" and "an" are used to refer to one or to over one (*i.e.,* to at least one) of the grammatical object of the article. For example, "an element" means one element or over one element.

The term "ammonia concentration" refers to an amount of ammonia dissolved in a given volume of a solvent.

A "ammonia sensor" can be any component or set of components capable of determining a concentration of ammonia within a fluid. In certain embodiments an ammonia sensor can determine both an ammonia and an ammonium ion concentration in a fluid.

The term "brine source" refers to a source of a solution of salts and/or buffers containing solutes used in recharging a sorbent material. In certain embodiments, the brine source can contain a sodium salt, acetic acid, sodium acetate, or combinations thereof.

The terms "communication" or "electronic communication" can refer to the ability to transmit electronic data, instructions, information wirelessly, via electrical connection, or any other electrical transmission between two components or systems.

The term "comparison" refers to a matching of a first value or first set of values with a second value or second set of values.

The term "comprising" includes, but is not limited to, whatever follows the word "comprising." Use of the term indicates the listed elements are required or mandatory but that other elements are optional and may be present.

The term "concentration" refers to an amount of a solute per a given volume of a solvent.

The term "consisting of' includes and is limited to whatever follows the phrase "consisting of." The phrase indicates the limited elements are required or mandatory and that no other elements may be present.

The term "consisting essentially of' includes whatever follows the term "consisting essentially of' and additional elements, structures, acts or features that do not affect the basic operation of the apparatus, structure or method described.

The term "dialysate flow path" can refer to a fluid pathway or passageway that conveys a fluid, such as dialysate and is configured to form at least part of a fluid circuit for peritoneal dialysis, hemodialysis, hemofiltration, hemodiafiltration or ultrafiltration.

The term "effluent" can refer to liquid, gas, or a combination thereof exiting a container, compartment, or cartridge.

An "effluent line" can be a fluid passageway, tube, or path of any kind into which liquid, gas, or a combination thereof exiting a container, module, or component can flow.

"Estimated," to "estimate," or "estimation" refer to a determination of one or more parameters indirectly using one or more variables.

The term "fluidly connectable" refers to the ability of providing for the passage of fluid, gas, or combination thereof, from one point to another point. The ability of providing such passage can be any connection, fastening, or forming between two points to permit the flow of fluid, gas, or combinations thereof. The two points can be within or between any one or more of compartments of any type, modules, systems, components, and rechargers.

The term "fluidly connected" refers to a particular state such that the passage of fluid, gas, or combination thereof, is provided from one point to another point. The connection state can also include an unconnected state, such that the two points are disconnected from each other to discontinue flow. It will be further understood that the two "fluidly connectable" points, as defined above, can from a "fluidly connected" state. The two points can be within or between any one or more of compartments, modules, systems, components, and rechargers, all of any type.

The term "inlet" can refer to a portion of a component through which fluid, gas, or a combination thereof can be drawn into the component.

The terms "introducing," "introduced," or to "introduce" refers to directionally moving or flowing a fluid, a gas, or a combination thereof by any means known to those of skill in the art.

A "known ammonia measurement curve" refers to a graphical or numeric representation of ammonia concentrations in a fluid over time for a given set of fluid parameters.

The term "outlet" can refer to a portion of a component through which fluid, gas, or a combination thereof can be drawn out of the component

A "patient" or "subject" is a member of any animal species, preferably a mammalian species, optionally a human. The subject can be an apparently healthy individual, an individual suffering from a disease, or an individual being treated for a disease. In certain embodiments, the patient can be a human, sheep, goat, dog, cat, mouse or any other animal.

The term "patient pre-dialysis blood urea nitrogen (BUN) level" can refer to the amount of nitrogen that comes from urea that is within the body of a patient prior to a dialysis session. The BUN measurement is generally given in units of mg/dl, but can also be given in units of millimoles per liter urea, or any other units of concentration.

The term "patient pre-dialysis solute level" can refer to the amount of a solute or set of solutes within the body of a patient prior to a dialysis session. The solute level measurements can be given in any units of concentration

The term "processor" as used is a broad term and is to be given its ordinary and customary meaning to a person of ordinary skill in the art. The term refers without limitation to a computer system, state machine, processor, or the like designed to perform arithmetic or logic operations using logic circuitry that responds to and processes the basic instructions that drive a computer. In any embodiment of the first, second, third, and fourth invention, the terms can include ROM ("read-only memory") and/or RAM ("random-access memory") associated therewith.

The term "programmed," when referring to a processor, can mean a series of instructions that cause a processor to perform certain steps. For example, a processor can be "programmed" to set functions, parameters, variables, or instructions.

The term "pump" refers to any device that causes the movement of fluids or gases by applying suction or pressure.

A "readable component" is any component that can contain information obtainable from a reader.

A "reader" is any component that can obtain information from a second component, such as a barcode or RFID component.

The terms "receiving," "to receive," or "received" in the context of data refers to obtaining information or any other means of data transmission or representation from any source by any means including direct electrical contact, induction, magnetic, wireless transmission, or networked connection.

A "recharge parameter" is any factor or variable used in recharging of a material. In certain embodiments, a recharge parameter can include one or more of a flow rate, concentration, or volume of recharge solutions used in recharging. Other non-limiting examples of a recharge parameter can be time of recharging, recharge temperature, volume of at least one recharge solution, concentration of at least one recharge solution, and combinations thereof.

A "recharge solution" is a solution containing appropriate ions for recharging a specific sorbent material. A recharge solution can be a single solution containing all necessary ions for recharging a sorbent material. Alternatively, the recharge solution can contain some of the ions for recharging the sorbent material, and one or more other recharge solutions can be used to recharge the sorbent material.

A "recharge solution source" is any fluid or concentrate source from which a recharge solution can be obtained.

The term "recharge temperature" refers to the temperature of one or more components used in recharging a sorbent material. The recharge temperature can refer to a temperature of one or more recharge solutions introduced through a sorbent module containing the sorbent material, or can refer to a temperature of the sorbent material itself.

"Recharging" refers to treating a sorbent material to restore the functional capacity of the sorbent material so as to put the sorbent material back into a condition for reuse or use in a new dialysis session. In some instances, the total mass, weight and/or amount of "rechargeable" sorbent materials remain the same. In some instances, the total mass, weight and/or amount of "rechargeable" sorbent materials change. Without being limited to any one theory of invention, the recharging process may involve exchanging ions bound to the sorbent material with different ions, which in some instances may increase or decrease the total mass of the system. However, the total amount of the sorbent material will in some instances be unchanged by the recharging process. Upon a sorbent material undergoing "recharging," the sorbent material can then be said to be "recharged."

A "recharging flow path" is a path through which fluid can travel while recharging sorbent material in a reusable sorbent module.

The term "sensor," as used herein, can be a converter of any type that can measure a physical property or quantity of a matter in a solution, liquid or gas, and can convert the measurement into a signal which can be read by an electronic instrument.

The term "setting," "set," or "to set" in the context of performing a series of instructions or steps refers to the process of adjusting or controlling one or more variable to a desired value for use in a process, method, or system.

The terms "set based at least in part on" or "set based on" refer to a calculation of a parameter value, wherein the value is a function of at least one other variable.

A "sorbent cartridge module" or "sorbent module" means a discreet component of a sorbent cartridge. Multiple sorbent cartridge modules can be fitted together to form a sorbent cartridge of two, three, or more sorbent cartridge modules. In some embodiments, a single sorbent cartridge module can contain all of the necessary materials for dialysis. In such cases, the sorbent cartridge module can be a "sorbent cartridge." The "sorbent cartridge module" or "sorbent module" can contain any material for use in sorbent dialysis and may or may not contain a "sorbent material" or adsorbent. In other words, the "sorbent cartridge module" or "sorbent module" generally refers to the use of the "sorbent cartridge module" or "sorbent module" in sorbent-based dialysis, e.g., REDY (REcirculating DYalysis), and not that a "sorbent material" is necessarily contained in the "sorbent cartridge module" or "sorbent module."

The term "time of recharging" refers to the total amount of time required to restore the functional capacity of a sorbent material for a given set of recharge parameters.

The term "volume" refers to a three-dimensional amount of space occupied by a material.

A "water source" is a fluid source from which water can be obtained.

"Zirconium phosphate" is a sorbent material that removes cations from a fluid, exchanging the removed cations for different cations.

*Zirconium Phosphate Recharging*

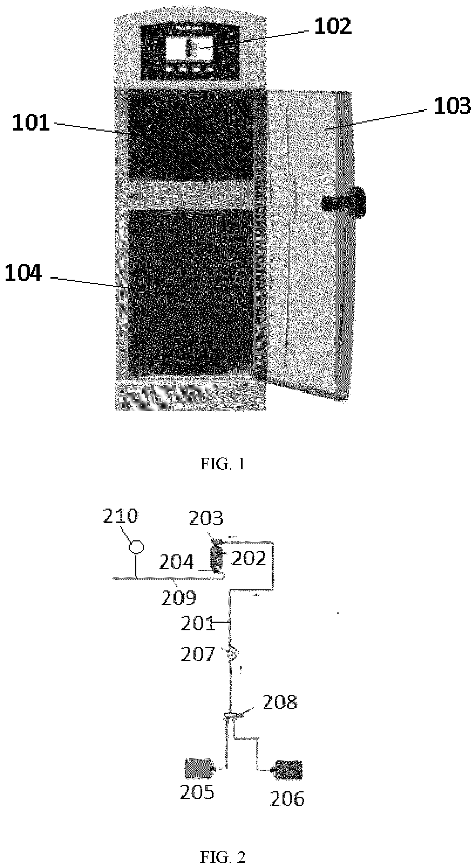

The invention is drawn to systems and methods for recharging and reusing zirconium phosphate in a reusable zirconium phosphate sorbent module. FIG. 1 illustrates a recharger for recharging zirconium phosphate in a zirconium phosphate sorbent module. The recharger includes at least a first receiving compartment **101** for receiving a zirconium phosphate sorbent module. The receiving compartment **101** has a sorbent module inlet and a sorbent module outlet (not shown) fluidly connectable to an inlet and outlet of a zirconium phosphate sorbent module (not shown). Door **103** controls access to the receiving compartment **101**. A user interface **102** can receive information from a user for controlling the recharge process. The recharger can optionally include a second receiving compartment **104** for receiving a second sorbent module, containing the same or a different sorbent material for concurrent recharging of sorbent materials. The recharger can include any number of receiving compartments for receiving multiple sorbent modules or various combinations of sorbent modules. The recharger can have 1, 2, 3, 4, 5, or more receiving compartments for recharging any number of sorbent modules. The recharger can be fluidly connectable to one or more recharge solution sources through a recharging flow path. Pumps and valves (not shown) control the movement of fluid from the recharge solution sources through the zirconium phosphate module.

Zirconium phosphate is recharged by introducing one or more solutions containing acids, bases, and sodium salts through the zirconium phosphate module. The hydrogen and sodium ions in the recharge solutions displace potassium, calcium, magnesium, ammonium, and other ions from either the dialysate or source water that are bound and adsorbed by the zirconium phosphate during use. The recharged zirconium phosphate with sodium and hydrogen ions can be used during dialysis to remove cation solutes from the used dialysate. As described, the amount of sodium and hydrogen ions required for recharging the zirconium phosphate can depend on the amount of ammonia produced from the breakdown of urea by urease in a sorbent cartridge, which in turn depends on the patient pre-dialysis blood urea nitrogen (BUN) level.

FIG. 2 illustrates a non-limiting embodiment of a zirconium phosphate recharging flow path 201 for recharging zirconium phosphate in a zirconium phosphate sorbent module 202. After dialysis, the zirconium phosphate sorbent module 202 can be removed from the dialysis system and placed in the sorbent recharger. The zirconium phosphate sorbent module 202 can be fluidly connectable to the zirconium phosphate recharging flow path 201 through zirconium phosphate sorbent module inlet 203 and zirconium phosphate sorbent module outlet 204. In certain embodiments, the direction of flow during recharging can be in the opposite direction as during therapy. That is, the zirconium phosphate sorbent module outlet 204 of the zirconium phosphate sorbent module 202 during recharging may be used as the inlet during dialysis therapy. The zirconium phosphate recharging flow path 201 can include at least one pump 207 to provide a driving force for moving fluids through the zirconium phosphate recharging flow path 201. In certain embodiments, two or more pumps can be included for individually introducing fluids from the recharge solution sources into the zirconium phosphate sorbent module 202. As described, the system and methods allow for setting a one or more recharge parameters used when introducing recharge solutions through the zirconium phosphate recharging flow path 201 for precision recharging. The zirconium phosphate recharging flow path 201 can include one or more recharge solution sources, including a brine source 205 and a water source 206. The brine source 205 can contain a brine solution of a salt, such as sodium chloride, and a buffer, such as a mixture of sodium acetate and acetic acid. The sodium and hydrogen ions in the recharge solution displace the ammonium ions and other cations adsorbed by the zirconium phosphate during a prior dialysis session. Although shown as a single brine source 205, multiple recharge solution sources can be used. For example, a first recharge solution source containing sodium chloride and a second recharge solution source containing an acetate buffer. Alternatively, three recharge solution sources can be used, with sodium chloride, sodium acetate, and acetic acid in separate recharge solution sources. If multiple recharge solution sources are used, the recharge solutions can be mixed within the zirconium phosphate recharging flow path 201 or pumped through the zirconium phosphate sorbent module 202 sequentially. Any combination of sodium salt and buffer capable of causing exchange of ammonium, potassium, calcium, and magnesium for sodium and hydrogen ions can be used as the recharge solutions. The water from water source 206 can be used to dilute the brine solution from brine source 205 if a concentrated brine solution is used, and to rinse the zirconium phosphate sorbent module 202 before and after introducing the brine solution through the zirconium phosphate sorbent module 202. A concentrated brine solution that is diluted with water allows a processor to set the concentration of the brine solution rather than using a pre-set concentration by controlling the relative flow rates of water and concentrated brine solution into the zirconium phosphate recharging flow path 201. In certain embodiments, the brine source 205 can contain a brine concentrate having a concentration of salt and buffer greater than that to be used in recharging. The brine solution can be diluted in-line with water from the water source 206 to generate a recharge solution with a desired concentration, as described. Optional valve 208 can be included to control the movement of fluid from either the brine source 205 or water source 206. Alternatively, separate pumps on fluid lines fluidly connected to each recharge solution source can be used. A processor (not shown) can be programmed to control the pumps or valves to direct recharge solutions from the recharge solution sources through the zirconium phosphate sorbent module 202 to recharge the zirconium phosphate with specified recharge parameters. The processor can receive or derive the patient pre-dialysis BUN level and set the recharge parameters used for recharging the zirconium phosphate. One of skill in the art will understand that multiple pump and valve arrangements can be used to pump the necessary recharge solutions through the zirconium phosphate sorbent module 202.

During a dialysis session, the zirconium phosphate can remove cations from spent dialysate, including ammonium, potassium, calcium, and magnesium, exchanging the cations for hydrogen and sodium ions. The ammonia is formed by the breakdown of urea by urease in the sorbent cartridge during treatment. The sodium chloride and buffer solutions used in recharging the zirconium phosphate serve to displace the cations absorbed during treatment with sodium and hydrogen ions, facilitating reuse of the zirconium phosphate.

An optional ammonia sensor 210 can be placed in effluent line 209 to measure the ammonia concentration of effluent exiting the zirconium phosphate sorbent module 202 during recharging. Displaced ammonia from the zirconium phosphate sorbent module 202 will exit the zirconium phosphate sorbent module 202 through zirconium phosphate sorbent module outlet 204 into effluent line 209. Ammonia sensor 210 can determine the total ammonia content of the effluent recharge solution.

One of skill in the art will understand that several methods can be used to determine the total ammonia content in the effluent line 209. In certain embodiments, the ammonia sensor 210 can determine concentrations of both ammonia and ammonium ions in the effluent line 209. Alternatively, the ammonia sensor 210 can determine either the ammonia or ammonium ion concentration and the pH, allowing the total ammonia content to be determined using the Henderson-Hasselbach equation. In certain embodiments, the ammonia sensor **210** can measure the partial pressure of ammonia gas, with the total ammonia content of the effluent determined using Henry's law and the Henderson-Hasselbach equation. Additional sensors, such as a temperature sensor can also be used. Although shown as a single ammonia sensor **210** in FIG. 2, the ammonia sensor **210** can alternatively be multiple sensors that determine individual parameters of the effluent recharge solution to allow for calculation of the total ammonia content in the effluent recharge solution. The ammonia sensor **210** can be in communication with a processor (not shown) programmed to estimate the patient pre-dialysis BUN level based on data received from the ammonia sensor **210.**

The processor can be programmed to receive the total ammonia content of the recharge solution effluent a single time or multiple times near the beginning of the recharging process and can compare the total ammonium content of the recharge solution effluent with known ammonia measurement curves or characterized ammonia measurement curves using a lookup table or other method of comparison. The flow rate of the recharge solution introduced through the zirconium phosphate sorbent module **202** can also be received by the processor. Based on the flow rate and ammonia concentration in the effluent line **209,** the processor can determine or estimate the patient pre-dialysis BUN level based on a comparison to the known or characterized ammonia measurement curves. In certain embodiments, the ammonia sensor **210** can be used in conjunction with the processor to determine the total ammonia content in the zirconium phosphate sorbent module **202** based on integration calculations given discrete time based measurements provided by the ammonia sensor **210.**

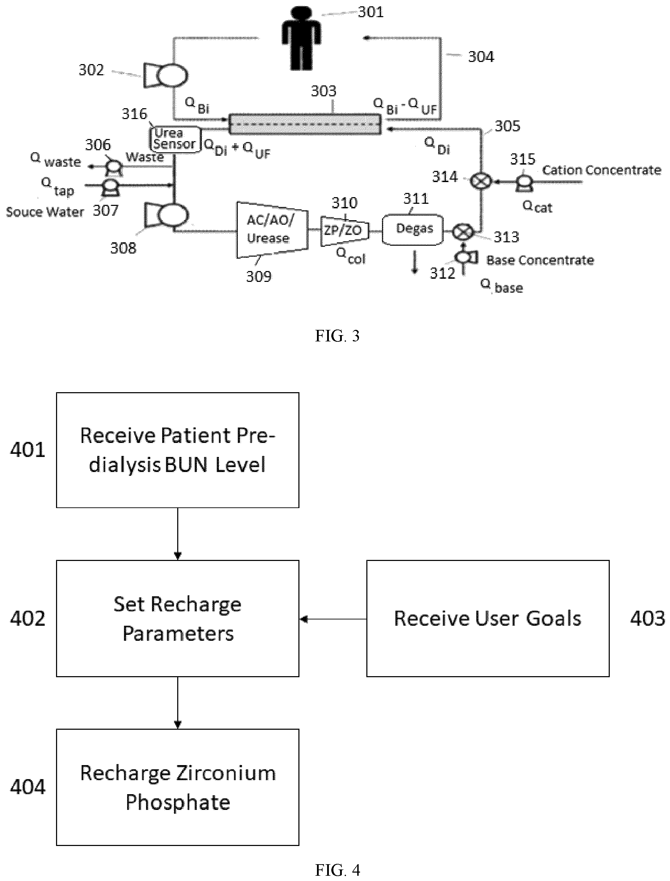

Alternative methods of obtaining the patient pre-dialysis BUN level can also be used, including methods during dialysis treatment. FIG. 3 illustrates a dialysis system using a single urea sensor **316** to obtain the patient pre-dialysis BUN level. The dialysis system includes an extracorporeal flow path **304** fluidly connected to a dialyzer **303**. Blood from the patient **301** is pumped through the extracorporeal flow path **304** by blood pump **302**. Dialysate is pumped through dialysate flow path **305** by dialysate pump **308**. Waste or ultrafiltrate can be removed by waste pump **306** at a flow rate of Qwaste. Additional water can be added to the dialysate flow path **305** by water pump **307** at a flow rate of Qtap. The dialysate regeneration system can include a first urease module **309** containing urease, and optionally activated carbon and/or alumina oxide, and a second zirconium phosphate module **310** containing zirconium phosphate and zirconium oxide. The flow rate of fluid through the dialysate regeneration system is given as Qcol. A urea sensor **316** determines the urea concentration in the dialysate upstream of the dialysate regeneration system. A degasser **311** can remove carbon dioxide formed from the breakdown of urea. Bicarbonate can be added from a bicarbonate source (not shown) by bicarbonate pump **312** at a bicarbonate addition rate of Qbase. A static mixer **313** can optionally be included to ensure complete mixing of the bicarbonate concentrate with the dialysate. Cation infusates, such as calcium, magnesium, and potassium can be added by cation concentrate pump **315** at a flow rate of Qcat. A static mixer **314** can optionally be included to ensure complete mixing of the cation concentrate with the dialysate. The flow rate of blood entering the dialyzer **303** in FIG. 3 is given as $Q_{Bi}$. The flow rate of blood exiting the dialyzer **303** will be $Q_{Bi} - Q_{uf}$, where $Q_{uf}$ denotes the ultrafiltration rate. The flow rate of dialysate entering the dialyzer **303** is given as $Q_{Di}$. The flow rate of dialysate exiting the dialyzer **303** will be $Q_{Di} + Q_{uf}$. One of skill in the art will understand that, using the flow rates described and the dialysate urea concentration as obtained from the urea sensor **316,** the patient pre-dialysis BUN level can be determined. As described, the determined patient pre-dialysis BUN level can be used to determine the amount of recharge solutions necessary for recharging the zirconium phosphate sorbent module **310**. Alternatively, ammonia, ammonium, and/or pH sensors positioned between the urease module **309** and zirconium phosphate sorbent module **310** can be used to estimate the patient pre-dialysis BUN level. US Patent Application No. 16/148,383 describes methods for estimating the patient pre-dialysis BUN level from the described sensors, and the entire contents thereof are hereby incorporated by reference.

Any alternative method of receiving the patient pre-dialysis BUN level can also be used for precision recharging, including blood draws and analysis, or estimations based on historical trends for the patient. The processor of the recharger can receive the patient pre-dialysis BUN levels and set the recharge parameters as described. Certain described methods of receiving the patient pre-dialysis BUN level include actual measurements of the urea in the dialysate, which provides an actual determination of the amount of urea processed by the sorbent cartridge. Other methods include estimating the patient pre-dialysis BUN level and then estimating the amount of urea processed by the sorbent cartridge. In either case, the system and methods described allow for precision recharging based on the amount of urea processed by the sorbent cartridge, either by direct determination or estimation.

In certain embodiments, usage of a zirconium phosphate sorbent module by a patient can be tracked with an RFID tag, barcode, or other readable component. Alternatively, the usage of the zirconium phosphate sorbent module can be sent via electronic or radio communication between the recharger and the dialysis therapy system. The processor can receive the patient pre-dialysis BUN level from the readable component with a reader in communication with the processor. A readable component, such as an RFID tag or bar code, can be affixed to the sorbent modules, and automatically read by the processor at various times, including prior to dialysis, after dialysis, prior to recharging, and after recharging. A single reader can read and track the sorbent modules at each stage of use, or separate readers can be included with the rechargers and dialysis systems to track usage of the sorbent modules. The tracking system can track which patients used the sorbent modules and the patient pre-dialysis BUN level. The patient pre-dialysis BUN level can be communicated to the processor, which can then determine the amount of recharge solution necessary through mathematical algorithms, look-up tables or a combination thereof.

Once the patient pre-dialysis BUN level has been obtained, the processor can set one or more recharge parameters for recharging the zirconium phosphate in the reusable zirconium phosphate sorbent module. The amount of sodium and hydrogen ions required to recharge the zirconium phosphate depends in part on the amount of ammonia adsorbed by the zirconium phosphate during treatment. EQ(1) provides an algorithm for setting a volume of a recharge solution required to recharge the zirconium phosphate.

$$\text{volume} = A * \text{Pre-BUN} + B \qquad\qquad EQ(1)$$

The volume in EQ (1) refers to the volume of the recharge solution needed for recharging. Pre-BUN refers to the patient pre-dialysis BUN level. As illustrated in EQ.'s (2-12), A and B depend on the concentration of the recharge solution, other recharge parameters such as flow rate and recharge temperature, estimates of the patient pre-dialysis solute levels of cations such as potassium, calcium, and magnesium, and dialysis session parameters like flow rate, dialyzer type and size, and session time. The A factor also accounts for bicarbonate generated from urea breakdown that is passed through the zirconium phosphate layer.

The volume of recharge solutions necessary for recharging the zirconium phosphate is based on the amount of ions adsorbed by the zirconium phosphate during treatment and can be calculated using EQ(1).

The total amount of urea introduced through the zirconium phosphate sorbent module during treatment is provided by EQ(2).

$$\text{Total urea} = Q_d * t * \bar{C}_{\text{urea}} \qquad\qquad EQ(2)$$

Where $Q_d$ is the average dialysate flow rate, t is the time of the dialysis session, and $C_{\text{urea}}$ is the average urea concentration in the dialysate. Alternatively, the total amount of urea introduced through the zirconium phosphate sorbent module during treatment is provided by EQ(3).

$$\text{Total urea} = V_{\text{prp}} * C_{\text{Bprp}} - V_{\text{post}} * C_{\text{Bpost}} \qquad\qquad EQ(3)$$

Where $= V_{\text{prp}}$ is the patient water volume prior to dialysis, $C_{\text{Bprp}}$ is the patient urea level prior to dialysis, $V_{\text{post}}$ is the patient water volume after dialysis, and $C_{\text{Bpost}}$ is the patient urea level after dialysis. The patient urea level after dialysis can be determined by EQ(4).

$$C_{\text{Bpost}} = URR * C_{\text{Bprp}} \qquad\qquad EQ(4)$$

Where URR is the urea reduction ratio for the dialysis treatment. Combining EQ's (2-3) gives EQ(5).

$$Q_d * t * \bar{C}_{\text{urea}} = V_{\text{prp}} * C_{\text{Bprp}} - V_{\text{post}} * URR * C_{\text{Bprp}} \qquad\qquad EQ(5)$$

EQ(5) can be solved for the average urea concentration in the dialysate during treatment to give EQ(6).

$$\bar{C}_{\text{urea}} = \frac{C_{Bprp}(V_{prp} - V_{post}*URR)}{Q_d * t} \qquad\qquad EQ(6)$$

Because each molecule of urea is converted to two ammonium ions by the urease, the total amount of urea introduced to the zirconium phosphate is two times the amount of urea introduced to the sorbent cartridge, shown in EQ(7).

$$\bar{C}_{\text{NH4}} = \frac{2 * C_{Bprp}(V_{prp} - V_{post}*URR)}{Q_d * t} \qquad\qquad EQ(7)$$

During treatment, bicarbonate is also introduced to the zirconium phosphate. The bicarbonate comes from both bicarbonate in the dialysate, and bicarbonate generated by the breakdown of urea by urease. EQ (8) provides the average concentration of bicarbonate entering the zirconium phosphate.

$$\bar{C}_{HCO3} = \bar{C}_{D, HCO3} + X * \bar{C}_{urea} \qquad\qquad EQ(8)$$

Where $\bar{C}_{HCO3}$ is the average bicarbonate concentration entering the zirconium phosphate, $\bar{C}_{D, HCO3}$ is the average dialysate bicarbonate concentration, and X is a measurement of the amount of bicarbonate generated by the breakdown of urea.

The total amount of recharge solution necessary for recharging is provided by EQ (9).

$$V_r = v * Q * t\,[2\bar{C}_{urea} + \bar{C}_K + \bar{C}_{Ca} + \bar{C}_{Mg} + (y * (\bar{C}_{D, HCO3} + X * \bar{C}_{urea}) + z]\qquad EQ(9)$$

Where Vr is the volume of recharge solution necessary to recharge the zirconium phosphate, Q is the time averaged volume flow rate into the zirconium phosphate sorbent module, t is the session time, $C_{NH4}$, $C_K$, $C_{Ca}$, $C_{Mg}$, and $C_{HCO3}$ are average concentrations of ammonium ions, potassium ions, calcium ions, magnesium ions and bicarbonate ions entering the zirconium phosphate sorbent module, y and z are variables related to the pH of the zirconium phosphate and relate to an amount of hydrogen released from the zirconium phosphate, and v is a variable specific to the recharge process being used. Rearranging EQ(9) provides EQ's (10-11).

$$V_r = 2\bar{C}_{urea} * v * Q * t + v * Q * t * y * \bar{C}_{D, HCO3} + v * Q * t * y * X * \bar{C}_{urea} + v * Q * t * y * (\bar{C}_K$$

$$+ \bar{C}_{Ca} + \bar{C}_{Mg} + z) \qquad\qquad EQ(10)$$

$$V_r = \bar{C}_{urea}\,(2 * X * v * Q * t + y * X * v * Q * t) + v * Q * t\,(\bar{C}_K + \bar{C}_{Ca} + \bar{C}_{Mg} + z + y * \bar{C}_{D, HCO3})$$

$$EQ\,(11)$$

Combining EQ(11) with EQ(7) gives EQ(12).

$$Vr = C_{Bprp}\,[\frac{2*X*(V_{prp}-V_{post}*URR)}{Q*t}\,(\,2X * v * Q* t + y * x * v * Q * t)] + v * Q * t\,(\bar{C}_K + \bar{C}_{Ca} + \bar{C}_{Mg}$$

$$+ z + y * \bar{C}_{D, HCO3}) \qquad\qquad EQ(12)$$

As described, the volume of recharge solution necessary is given by EQ(1), where the A factor is $\frac{2*X*(V_{prp}-V_{post}*URR)}{Q*t}\,(\,2X * v * Q* t + y * x * v * Q * t)$ and the B factor is $v * Q * t(\bar{C}_K + \bar{C}_{Ca} + \bar{C}_{Mg} + z + y * \bar{C}_{D, HCO3})$.

To avoid the need for determining the average concentrations of all cations in the dialysate, in certain embodiments, the concentrations of potassium, calcium, and magnesium can be estimated. The estimation can be based on a prior history of the patient. Alternatively, the estimates of the concentrations of potassium, calcium, and magnesium can be set based on a "worst-case scenario," or at the maximum values that might be expected in the patient. By setting the concentrations at the maximum values, complete recharging is ensured, while reducing the total volume of recharge solution used based on the actual or estimated patient pre-dialysis BUN level.

One of skill in the art will understand that there are interdependencies between concentration, recharge temperature, time of recharging, volume and flow rate. For example, at a faster flow rate or at a higher recharge solution concentration, the time of recharging may be lower, but may require additional chemicals. At lower flow rates or recharge solution concentrations the process can be more efficient with respect to chemical usage, but can take longer. A user can input the values that are desired to be minimized based on the user objectives, and the system can determine the proper concentration, flow rate, and/or volume of the recharge solutions to minimize the desired parameters based on the patient pre-dialysis BUN level. The system can be adaptive to the needs of the user, with the user defining the desired objectives for the recharging process, such as to minimize time or to minimize chemical usage In certain embodiments, the sorbent recharger can use a higher concentration or flow rate at the beginning of recharging, and adjust the concentration and/or flow rate lower as the recharging process nears completion, minimizing both the time and volume of recharge solutions necessary for recharging the zirconium phosphate.

FIG. 4 illustrates a flow chart for precision recharging of zirconium phosphate in a zirconium phosphate sorbent module based on the patient pre-dialysis BUN level. In step 401, the system can receive the pre-dialysis BUN level of the patient that used the zirconium phosphate sorbent module. As described, the processor can receive the patient pre-dialysis BUN level from any source. In certain embodiments, a readable component, such as an RFID tag or bar code, can be affixed to or embedded in the sorbent modules, and automatically read by a reader in communication with the processor, including prior to dialysis, after dialysis, prior to recharging, and after recharging. The reader can track the sorbent modules and patients that used the sorbent modules and contain the patient pre-dialysis BUN level. The patient pre-dialysis BUN level can be communicated to the processor, which can then determine the amount of recharge solution necessary through mathematical algorithms, look-up tables or a combination thereof. Alternatively, a user can enter the patient pre-dialysis BUN level through a user interface in communication with the processor. The user can obtain the patient pre-dialysis BUN level from blood draws and analysis or any other source. Alternatively, the patient pre-dialysis BUN level can be estimated based on a prior history of the patient, using a determined patient pre-dialysis BUN level from a prior session, or an average of prior sessions. In certain embodiments, the processor can determine the patient pre-dialysis BUN level based on an ammonia sensor in an effluent line fluidly connected to the zirconium phosphate sorbent module. In such embodiments, the recharging process can begin using a predetermined set of recharge parameters, and the processor can alter the recharge parameters after determining the patient pre-dialysis BUN level.

Optionally, in step 403, user goals can be received by the system. As described, the user can request that the recharging take a certain amount of time, or use a certain amount of chemicals. In step 402, the system can set the recharge parameters based on the patient pre-dialysis BUN level, and optionally the user goals, such as to minimize time, chemicals, or any other factors. In step 404, the system can introduce the recharge solutions into and through the sorbent module using the recharge parameters set in step 402 to recharge the sorbent material inside the sorbent module.

The processor can set the recharge parameters using any method known in the art, including lookup tables, mathematical algorithms, or a combination thereof. For example, the processor can use a lookup table to determine the proper recharge parameters based on a given patient pre-dialysis BUN level. The processor can also use lookup tables or mathematical algorithms to adjust one or more recharge parameters based on user goals, as described. For example, if a user wishes to minimize the amount of brine solution used in recharging zirconium phosphate, the processor can then set a lower flow rate and brine concentration and a higher recharge temperature based on the lookup tables. In certain embodiments, the lookup tables can include specific recharge parameters based on the received patient pre-dialysis BUN level. Alternatively, options can be provided based on the patient pre-dialysis BUN level, and the user can select the desired option based on the user goals.

One skilled in the art will understand that various combinations and/or modifications and variations can be made in the described systems and methods depending upon the specific needs for operation. Moreover, features illustrated or described as being part of an aspect of the invention may be used in the aspect of the invention, either alone or in combination, or follow a preferred arrangement of one or more of the described elements.

The invention may be described by reference to the following numbered paragraphs:

1. A system comprising: a recharging flow path comprising one or more recharge solution sources; the one or more recharge solution sources fluidly connectable to an inlet of a zirconium phosphate sorbent module containing zirconium phosphate; and at least one pump for introducing one or more recharge solutions from the one or more recharge solution sources through the zirconium phosphate sorbent module; and a processor, the processor programmed to set one or more recharge parameters to recharge the zirconium phosphate within the sorbent module based on a patient pre-dialysis BUN level.

2. The system of paragraph 1, wherein the one or more recharge solution sources comprise a brine source and a water source.

3. The system of paragraphs 1 or 2, wherein the one or more recharge parameters comprise at least one of: time of recharging, recharge temperature, volume of at least one recharge solution, concentration of at least one recharge solution, and combinations thereof.

4. The system of any of paragraphs 1-3, wherein the processor sets a volume of the one or more recharge solutions using an equation: Volume = A * Pre-BUN + B; wherein A and B are based on patient pre-dialysis solute level and a concentration of the one or more recharge solutions.

5. The system of any of paragraphs 1-3, wherein the processor is programmed to set the volume of the one or more recharge solutions based on the patient pre-dialysis BUN level and a concentration of the one or more recharge solutions.

6. The system of any of paragraphs 1-3, wherein the processor is programmed to set the concentration of the one or more recharge solutions based on the patient pre-dialysis BUN level, and a volume of the one or more recharge solutions to be used.

7. The system of any of paragraphs 1-6, further comprising at least one ammonia sensor in an effluent line fluidly connectable to an outlet of the zirconium phosphate sorbent module; wherein the processor is programmed to estimate the patient pre-dialysis BUN level based on an ammonia concentration in an effluent from the zirconium phosphate sorbent module.

8. The system of paragraph 7, wherein the processor is programmed to estimate the patient pre-dialysis BUN level based on a comparison of the ammonia concentration in the effluent with a known ammonia measurement curve.

9. The system of any of paragraphs 1-8, wherein the processor is programmed to receive a patient pre-dialysis BUN level from a user.

10. The system of any of paragraphs 1-6, further comprising a reader in communication with the processor, the reader receiving the patient pre-dialysis BUN level from a readable component on or in the sorbent module.

11. A method, comprising the steps of:

recharging zirconium phosphate within a zirconium phosphate sorbent module by introducing one or more recharge solutions from one or more recharge solution sources through the zirconium phosphate sorbent module with one or more recharge parameters; wherein the one or more recharge parameters are set based on a patient pre-dialysis BUN level.

12. The method of paragraph 11, wherein the one or more recharge parameters comprise at least one of: time of recharging, recharge temperature, volume of at least one recharge solution, concentration of at least one recharge solution, and combinations thereof.

13. The method of paragraphs 11 or 12, wherein the one or more recharge solution sources comprise a brine source and a water source.

14. The method of any of paragraphs 11-13, further comprising the step of estimating the patient pre-dialysis BUN level based on an ammonia sensor in an effluent line fluidly connected to an outlet of the zirconium phosphate sorbent module.

15. The method of any of paragraphs 11-13, further comprising the step of estimating the patient pre-dialysis BUN level based on one or more sensors in a dialysate flow path.

16. The method of any of paragraphs 11-13, further comprising the step of receiving the patient pre-dialysis BUN level from a user.

17. The method of any of paragraphs 11-16, wherein setting the recharge parameters comprises setting the concentration of the one or more recharge solutions based on the patient pre-dialysis BUN level and a volume of the one or more recharge solutions to be used.

18. The method of any of paragraphs 11-16, wherein setting the recharge parameters comprises setting the volume of the one or more recharge solutions based on the patient pre-dialysis BUN level and a concentration of the one or more recharge solutions.

19. The method of any of paragraphs 11-16, wherein setting the recharge parameters comprises setting the volume of the one or more recharge solutions using an equation volume = A * Pre-BUN + B; wherein A and B are based on patient pre-dialysis solute levels and a concentration of the one or more recharge solutions.

20. The method of any of paragraphs 11-19, wherein the method is performed by a processor of a sorbent recharger.

**Claims**

1. A system comprising:

a recharging flow path comprising one or more recharge solution sources; the one or more recharge solution sources fluidly connectable to an inlet of a zirconium phosphate sorbent module containing zirconium phosphate; and at least one pump for introducing one or more recharge solutions from the one or more recharge solution sources through the zirconium phosphate sorbent module; and a processor, the processor programmed to set one or more recharge parameters to recharge the zirconium phosphate within the sorbent module based on a patient pre-dialysis BUN level, preferably wherein the one or more recharge solution sources comprise a brine source and a water source.

2. The system of claim 1, wherein the one or more recharge parameters comprise at least one of: time of recharging, recharge temperature, volume of at least one recharge solution, concentration of at least one recharge solution, and combinations thereof.

3. The system of any of claims 1 or 2, wherein the processor sets a volume of the one or more recharge solutions using an equation: Volume = A * Pre-BUN + B; wherein A and B are based on patient pre-dialysis solute level and a concentration of the one or more recharge solutions.

4. The system of any of claims 1 or 2, wherein the processor is programmed to set the volume of the one or more recharge solutions based on the patient pre-dialysis BUN level and a concentration of the one or more recharge solutions.

5. The system of any of claims 1 or 2, wherein the processor is programmed to set the concentration of the one or more recharge solutions based on the patient pre-dialysis BUN level, and a volume of the one or more recharge solutions to be used.

6. The system of any of claims 1-5, further comprising at least one ammonia sensor in an effluent line fluidly connectable to an outlet of the zirconium phosphate sorbent module; wherein the processor is programmed to estimate the patient pre-dialysis BUN level based on an ammonia concentration in an effluent from the zirconium phosphate sorbent module.

7. The system of claim 6, wherein the processor is programmed to estimate the patient pre-dialysis BUN level based on a comparison of the ammonia concentration in the effluent with a known ammonia measurement curve.

8. The system of any of claims 1-7, wherein the processor is programmed to receive a patient pre-dialysis BUN level from a user.

9. The system of any of claims 1-5, further comprising a reader in communication with the processor, the reader receiving the patient pre-dialysis BUN level from a readable component on or in the sorbent module.

10. A method, comprising the steps of:

recharging zirconium phosphate within a zirconium phosphate sorbent module by introducing one or more recharge solutions from one or more recharge solution sources through the zirconium phosphate sorbent module with one or more recharge parameters;
wherein the one or more recharge parameters are set based on a patient pre-dialysis BUN level, preferably wherein the one or more recharge solution sources comprise a brine source and a water source;

optionally further comprising the step of estimating the patient pre-dialysis BUN level based on an ammonia sensor in an effluent line fluidly connected to an outlet of the zirconium phosphate sorbent module;
optionally further comprising the step of estimating the patient pre-dialysis BUN level based on one or more sensors in a dialysate flow path;
optionally further comprising the step of receiving the patient pre-dialysis BUN level from a user.

11. The method of claim 10, wherein the one or more recharge parameters comprise at least one of: time of recharging, recharge temperature, volume of at least one recharge solution, concentration of at least one recharge solution, and combinations thereof.

12. The method of any of claims 10-11, wherein setting the recharge parameters comprises setting the concentration of the one or more recharge solutions based on the patient pre-dialysis BUN level and a volume of the one or more recharge solutions to be used.

13. The method of any of claims 10-11, wherein setting the recharge parameters comprises setting the volume of the one or more recharge solutions based on the patient pre-dialysis BUN level and a concentration of the one or more recharge solutions.

14. The method of any of claims 10-11, wherein setting the recharge parameters comprises setting the volume of the one or more recharge solutions using an equation volume = A * Pre-BUN + B; wherein A and B are based on patient pre-dialysis solute levels and a concentration of the one or more recharge solutions.

15. The method of any of claims 10-14, wherein the method is performed by a processor of a sorbent recharger.

FIG. 1

FIG. 2

FIG. 3

FIG. 4

Europäisches
Patentamt

European
Patent Office

Office européen
des brevets

# EUROPEAN SEARCH REPORT

**Application Number**

EP 20 16 4524

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/221852 A1 (PUDIL BRYANT J [US] ET AL) 9 August 2018 (2018-08-09) * abstract; figures 1,4 * * paragraphs [0140] - [0150] * ----- | 1-15 | INV. A61M1/16 A61M1/36 B01J20/34 |
| X | US 2017/087533 A1 (HOBOT CHRISTOPHER M [US] ET AL) 30 March 2017 (2017-03-30) * abstract; figures 1,4 * * paragraphs [0092] - [0098] * ----- | 1-15 | |
| E | EP 3 626 280 A1 (MEDTRONIC INC [US]) 25 March 2020 (2020-03-25) * abstract; figures * * paragraph [0101] * ----- | 1,2,4-8, 10-12,15 | |
| A | US 2015/367056 A1 (GERBER MARTIN T [US] ET AL) 24 December 2015 (2015-12-24) * abstract; figures * * paragraphs [0148], [0150] - [0152] * ----- | 1,10 | |
| | | | TECHNICAL FIELDS SEARCHED (IPC) A61M G01N B01J |

The present search report has been drawn up for all claims

| Place of search | Date of completion of the search | Examiner |
|---|---|---|
| The Hague | 5 August 2020 | Kaden, Malte |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 16 4524

This annex lists the patent family members relating to the patent documents cited in the above-mentioned European search report.
The members are as contained in the European Patent Office EDP file on
The European Patent Office is in no way liable for these particulars which are merely given for the purpose of information.

05-08-2020

| Patent document cited in search report | Publication date | Patent family member(s) | Publication date |
|---|---|---|---|
| US 2018221852 A1 | 09-08-2018 | US 2018221852 A1<br>US 2020156044 A1 | 09-08-2018<br>21-05-2020 |
| US 2017087533 A1 | 30-03-2017 | NONE | |
| EP 3626280 A1 | 25-03-2020 | CN 110917660 A<br>EP 3626280 A1<br>US 2020086297 A1 | 27-03-2020<br>25-03-2020<br>19-03-2020 |
| US 2015367056 A1 | 24-12-2015 | EP 3160535 A1<br>US 2015367056 A1<br>US 2019099538 A1<br>WO 2015199766 A1 | 03-05-2017<br>24-12-2015<br>04-04-2019<br>30-12-2015 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 14838318
- US 62583356
- US 87236318
- US 62456700
- US 148383